# EUROPÄISCHE PATENTANMELDUNG

(11) **EP 1 972 355 A1**
(43) Veröffentlichungstag der Anmeldung: **24.09.2008**
(21) Anmeldenummer: 08004896.0
(22) Anmeldetag: 15.03.2008
(51) Int. Cl.: A61M 5/28, B29C 49/06

(54) **Verfahren zur Herstellung einer Mehrkammer-Spritze mit Bypasskanal**

(30) Priorität: 19.03.2007 DE 102007014281
(71) Anmelder: Schott AG, 55122 Mainz (DE)
(72) Erfinder: Moesli, Thomas, 9014 St. Gallen (CH); Dudler, Raffael, 9000 St. Gallen (CH); Wolbring, Peter, Dr., 66606 St. Wendel (CH)
(74) Vertreter: Gahlert, Stefan

(57) **Zusammenfassung**

Ein Verfahren zur Herstellung einer Mehrkammer-Spritze (10) mit Bypasskanal (22) aus Kunststoff umfasst die Schritte: Herstellen eines rohrförmigen Vorformings (36) aus einem thermoformbaren Kunststoff; Bereitstellen einer Matrize (38), die zum Umschließen des Vorformlings (36) von außen in einem Bereich des zu bildenden Bypasskanals (22) ausgebildet ist, wobei der zumindest eine Bypasskanal (22) als Vertiefung (44,46) an einer Innenoberfläche (48,50) der Matrize (38) ausgebildet ist; Erwärmen des Vorformlings (36) bis auf eine Temperatur oberhalb des Erweichungsbereiches des Kunststoffes und Platzieren in einem Bereich innerhalb der Matrize (38) an einer vorbestimmten axialen Position; Beaufschlagen des Vorformlings (36) mit einer Druckdifferenz, bis der mindestens eine Bypasskanal (22) plastisch ausgeformt ist und Abkühlen des so hergestellten Spritzenkörpers auf Raumtemperatur.

## Beschreibung

Die Erfindung betrifft ein Verfahren zur Herstellung einer Mehrkammer-Spritze mit Bypasskanal aus Kunststoff.

Mehrkammer-Fertigspritzen werden in der Medizin zur Verabreichung von Präparaten eingesetzt, die aus mehreren Komponenten bestehen. In der Regel handelt es sich um zwei Komponenten, die entweder in der Kombination flüssig/flüssig oder in der Kombination fest/flüssig vor Verabreichung in der Spritze miteinander gemischt werden. Derartige Spritzen haben den Vorteil, dass das Mischen der Komponenten ohne Umfüllen in ein anderes Behältnis erfolgen kann und dass aus dem Behältnis heraus dann direkt eine Verabreichung erfolgen kann. Eine Zweikammer-Spritze weist einen rohrförmigen, im Allgemeinen zylindrischen Raum auf, der durch ein Trennelement aus einem elastomeren Material in eine erste Kammer und in eine zweite Kammer getrennt ist. Im Bereich der ersten Kammer, die in einer Ausflussöffnung mündet, ist ein Bypasskanal vorgesehen. Wird eine Substanz, die sich in der zweiten Kammer befindet, durch Betätigen eines Kolbens, durch den die zweite Kammer nach außen abgeschlossen ist, in Richtung auf die erste Kammer verschoben, so bewegt sich mit dem Kolben auch das Trennelement, bis dieses zunächst in den Bereich des Bypasskanals gelangt. In dieser Stellung ist ein Überströmen zwischen der ersten Kammer und der zweiten Kammer über den Bypasskanal ermöglicht, so dass eine Durchmischung der Substanzen stattfindet. Der Vorgang des Zusammenführens der beiden Substanzen ist abgeschlossen, wenn das Dichtungselement des Kolbens auf das Trennelement stößt. Danach können beide Dichtelemente zur Entleerung der Spritze bis an das Ausflussende des Spritzenzylinders zusammen verschoben werden.

Im Stand der Technik sind eine Reihe von Zweikammer-Spritzen bekannt, die etwa aus Glas bestehen (vgl. US 4 469 482) oder aber aus Kunststoff (vgl. EP 0 718 002 B1 und EP 0 556 034 A1).

Während die Herstellung von Mehrkammer-Spritzen aus Glas infolge der aufwändigen Verarbeitung relativ teuer ist, ist eine Zweikammer-Spritze gemäß der EP 0 718 002 B1 aus zwei Teilzylindern zusammengesetzt, die zunächst getrennt voneinander befüllt werden und erst dann zusammengefügt werden. Hierdurch lässt sich zwar das Risiko der so genannten Cross-Kontamination verringern, jedoch ist das Herstellverfahren relativ aufwändig und teuer.

Bei der aus der EP 0 556 034 A1 bekannten Zweikammer-Spritze wird diese durch Spritzgießen aus einem Stück hergestellt. Allerdings ist dieser Schrift nicht entnehmbar, wie der Bypasskanal erzeugt wird.

Es ist im Stand der Technik grundsätzlich bekannt, Hinterschnitte im Innenraum von Spritzgussteilen mithilfe von so genannten Faltkernsystemen zu erzeugen (vgl. hierzu DE 29 25 858 A1).

Die Verwendung solcher Faltkernsysteme ist allerdings sehr kosten- und pflegeintensiv und häufig nicht ausreichend zuverlässig. Für kleine Spritzenformate lassen sich Faltkernsysteme nicht einsetzen, da der Raum fehlt, die Faltmechanik im Kern unterzubringen. Auch sind die Fertigungskosten und Werkzeugkosten erheblich höher als bei Spritzenkörpern ohne Bypasskanal.

Der Erfindung liegt somit die Aufgabe zugrunde, ein Verfahren zur Herstellung einer Mehrkammer-Spritze mit Bypasskanal aus Kunststoff anzugeben, mit dem eine einfache und kostengünstige Herstellung in großen Stückzahlen ermöglicht ist.

Diese Aufgabe wird erfindungsgemäß durch ein Verfahren zur Herstellung einer Mehrkammer-Spritze mit Bypasskanal aus Kunststoff mit folgenden Schritten gelöst:
- Herstellen eines rohrförmigen Vorformlings, vorzugsweise durch Spritzgießen, aus einem thermoformbaren Kunststoff;
- Bereitstellen einer Matrize, die zur Umschließung des Vorformlings von außen in einem Bereich eines zu bildenden Bypasskanals ausgebildet ist, wobei der zumindest eine Bypasskanal als Vertiefung an einer Innenoberfläche der Matrize ausgebildet ist;
- Erwärmen des Vorformlings bis auf eine Temperatur oberhalb des Erweichungsbereiches, vorzugsweise innerhalb des Warmformbereiches, des Kunststoffes und Platzieren in einem Bereich innerhalb der Matrize an einer vorbestimmten axialen Position;
- Beaufschlagen des Vorformlings mit einer Druckdifferenz, bis der mindestens eine Bypasskanal plastisch ausgeformt ist; und
- Abkühlen des so hergestellten Spritzenkörpers auf Raumtemperatur.

Die Aufgabe der Erfindung wird auf diese Weise vollkommen gelöst.

Erfindungsgemäß wird zunächst der Vorformling in grundsätzlich bekannter Weise aus einem thermoformbaren Kunststoff, vorzugsweise durch Spritzgießen, hergestellt. Anschließend wird der Vorformling im Bereich des mindestens einen zu erzeugenden Bypasskanals unter Druck und Temperatur unter Zuhilfenahme einer Matrize verformt, so dass der mindestens eine Bypasskanal durch Fließen des Kunststoffmaterials bis an die Innenoberfläche der Matrize, in der der Bypasskanal als Vertiefung ausgebildet ist, plastisch ausgeformt wird. Auf diese Weise kann ohne Zuhilfenahme von Faltkernsystemen eine kostengünstige Herstellung einer Mehrkammer-Spritze aus Kunststoff erreicht werden. Das Verfahren ist ferner für hohe Stückzahlen geeignet.

In vorteilhafter Weiterbildung der Erfindung wird der Vorformling zur Ausformung des mindestens einen Bypasskanals mit einem Überdruck von innen beaufschlagt.

Hierzu kann bspw. ein Überdruck von mindestens 1 bar, vorzugsweise von mindestens 1,5 bar, weiter bevorzugt von mindestens 2 bar, vorzugsweise von mindestens 2,5 bar verwendet werden. Der verwendete Druck wird natürlich von dem verwendeten Kunststoffmaterial und der Temperatur, bei der der Fließvorgang erfolgt, beeinflusst.

Der maximale Druck während der Ausformung des Bypasskanals aus dem Vorformling ist durch die verwendete Apparatur begrenzt und liegt vorzugsweise bei etwa 10 bar, weiter bevorzugt bei höchstens 8 bar, weiter bevorzugt bei höchstens 5 bar, weiter bevorzugt bei höchstens 4 bar.

Die Temperatur, bei der die Ausformung des mindestens einen Bypasskanals erfolgt, hängt vom verwendeten Kunststoff ab und liegt je nach Kunststoffsorte vorzugsweise im Bereich zwischen 120 und 220°C, weiter bevorzugt zwischen etwa 140 und 200°C, weiter bevorzugt zwischen 160 und 180°C, besonders bevorzugt bei etwa 170°C, sofern etwa als Kunststoff ein lineares oder zyklisches olefinisches Polymer oder Copolymer verwendet wird.

Gemäß einer weiteren Ausgestaltung der Erfindung erfolgt die Beaufschlagung des erwärmten Vorformlings mit einer Druckdifferenz über eine Zeit zwischen 0,5 und 10 Sekunden, vorzugsweise zwischen 1 und 5 Sekunden, um mindestens einen Bypasskanal auszuformen, wobei bspw. eine Wandstärke des Vorformlings im Bereich des mindestens einen Bypasskanals zwischen 0,5 und 2 mm verwendet werden kann.

Gemäß einer weiteren Ausgestaltung der Erfindung wird der Vorformling zur Ausformung des mindestens einen Bypasskanals mit einem Unterdruck von außen beaufschlagt.

Hierbei lassen sich zwar keine so hohen Drücke wie bei der Ausformung durch Druckbeaufschlagung von innen erzielen, jedoch reicht auch der maximale Unterdruck von höchstens 1 bar aus, um bei Verwendung eines geeigneten Kunststoffes die Ausformung des mindestens einen Bypasskanals auf zuverlässige Weise zu erreichen.

Der Vorformling ist vorzugsweise im Wesentlichen zylindrisch ausgebildet und weist ein erstes Ende mit einer Verjüngung und ein zweites Ende auf, an dem vorzugsweise eine Handhabe, etwa in Form eines Flansches, angeformt ist.

Gemäß einer weiteren Ausgestaltung der Erfindung wird der Vorformling an seiner Innenoberfläche mit einem Gleitmaterial, vorzugsweise mit einem Silikonöl, beschichtet, bevor der mindestens eine Bypasskanal erzeugt wird.

Auf diese Weise ist eine einfache und zuverlässige Auftragung des Gleitmaterials auf die Innenoberfläche des Spritzenkörpers ermöglicht.

Nach der Herstellung des Spritzenkörpers und mit dem ausgeformten Bypasskanal bzw. den Bypasskanälen wird anschließend zweckmäßigerweise zunächst ein Trennelement in den Spritzenkörper derart eingeführt, dass eine erste Kammer auf der Seite des ersten Endes gebildet wird, die den mindestens einen Bypasskanal einschließt und durch das Trennelement von einer zweiten Kammer auf der Seite des zweiten Endes getrennt ist.

Die erste Kammer kann anschließend mit einem ersten Medium befüllt werden und durch Aufsetzen eines Verschlusses auf das erste Ende verschlossen werden, während die zweite Kammer mit einem zweiten Medium befüllt und mittels eines Kolbens verschlossen werden kann, an dem ein Dichtelement zur Abdichtung der zweiten Kammer vorgesehen ist.

Das Trennelement und das Dichtelement bestehen vorzugsweise aus einem elastomeren Material.

Die zur Ausformung des mindesten einen Bypasskanals verwendete Matrize ist vorzugsweise als zweiteilige Matrize ausgebildet, bestehend aus zwei Matrizenhälften.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der Erfindung zu verlassen.

Weitere Merkmale und Vorteile der Erfindung ergeben sich aus der nachfolgenden Beschreibung bevorzugter Ausführungsbeispiele unter Bezugnahme auf die Zeichnung. Es zeigen:
- Fig. 1: eine komplett montierte erfindungsgemäße Zweikammer-Spritze;
- Fig. 2: einen Längsschnitt durch einen Vorformling mit einer schematisch angedeuteten Matrize bestehend aus zwei Hälften, die von außen um die Außenoberfläche des Vorformlings aufgesetzt wird, um in einem nachfolgenden Bearbeitungsschritt unter Druck und Hitzeeinwirkung den oder die Bypasskanäle auszuformen und
- Fig. 3: einen vergrößerten Längsschnitt durch die beiden Matrizenhälften gemäß Fig. 2.

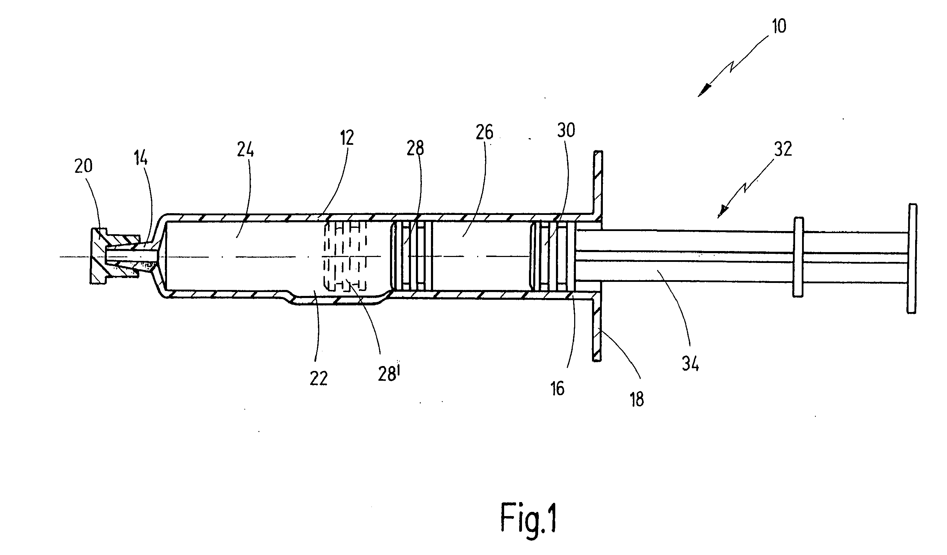

In Fig. 1 ist eine erfindungsgemäße Mehrkammer-Spritze im Längsschnitt dargestellt und insbesondere mit der Ziffer 10 bezeichnet.

Die Mehrkammer-Spritze 10 weist einen aus Kunststoff bestehenden Spritzenkörper 12 mit einem ersten Ende 14 und einem zweiten Ende 16 auf. Das erste Ende 14 ist als Auslaufspitze ausgebildet und mittels einer Verschlusskappe 20 verschlossen.

Es sind auch Ausführungen denkbar, bei denen eine Nadel unmittelbar am ersten Ende 14 festgelegt ist, z.B. eingeklebt ist oder mittels eines geeigneten Gewindes eingeschraubt ist.

Das zweite Ende 16 ist durch einen Injektionskolben 32 verschlossen, mit einer Kolbenstange 34, die durch ein Dichtelement 30 abgeschlossen ist und so den Raum innerhalb des Spritzenkörpers 12 nach außen abdichtet. Innerhalb des Spritzenkörpers 12 ist ferner ein Trennelement 28 in Form eines Stopfens aus einem elastomeren Material vorgesehen, durch das das Innere des Spritzenkörpers 12 in eine erste Kammer 24 und eine zweite Kammer 26 aufgeteilt wird. Die erste Kammer 24 ist nach außen durch das erste Ende 14 bzw. die aufgesetzte Verschlussskappe 20 begrenzt, während die zweite Kammer 26 nach außen durch das Dichtelement 30 des Injektionskolbens 32 abgeschlossen ist.

In einem mittleren Bereich des Spritzenkörpers 12 befindet sich unmittelbar angrenzend an das Trennelement 28 ein Bypasskanal 22, dessen axiale Ausdehnung länger ist als die axiale Ausdehnung des Trennelementes 28.

In die erste Kammer 24 kann bspw. ein erstes Pharmazeutikum eingefüllt sein und in die zweite Kammer 26 ein zweites Pharmazeutikum.

Zum Gebrauch wird die Spritze 10 mit einer Hand ergriffen, um den Injektionskolben 34 in den Spritzenkörper 12 einzuschieben, wobei eine Handhabe 18 am zweiten Ende 16, die als Fingerflansch ausgebildet ist, zum Ergreifen des Spritzenkörpers 12 dient. Hierbei erfolgt eine Entlüftung über das erste Ende 14. Wird der Injektionskolben 34 nunmehr nach innen geschoben, so wandert das Trennelement 28 in Richtung des ersten Endes 14, bis dieses in den Bereich des Bypasskanals 22 gelangt und hierdurch ein Überströmen zwischen der ersten Kammer 24 und der zweiten Kammer 26 ermöglicht ist. Wird der Injektionskolben 34 weiterbewegt, so gelangt das Dichtelement 30 schließlich zur Anlage an dem Trennelement 28, so dass beide Elemente 28, 30 gemeinsam weiterbewegt werden können. Zu diesem Zeitpunkt ist die Vermischung der beiden zuvor in den Kammern 24 und 26 aufgenommenen Substanzen abgeschlossen und die vermischten Komponenten können durch weiteres Einschieben des Injektionskolbens 34 injiziert werden.

Hierzu kann eine bereits vorhandene Injektionsnadel verwendet werden oder aber bspw. eine Injektionsnadel, die auf das erste Ende aufgeschraubt wird. Beliebige andere Ausführungen mit oder ohne Injektionsnadel sind denkbar. Form und Anordnung des mindestens einen Bypasskanals 22 können in weiten Grenzen variieren, wie grundsätzlich im Stand der Technik bekannt. So können bspw. mehrere Bypasskanäle 22 vorgesehen sein, die sich entlang der Innenumfangsfläche des Spritzenkörpers 12 in Axialrichtung oder schräg dazu erstrecken. Weiterhin können bspw. Y-förmige Bypasskanäle oder S-förmige Bypasskanäle vorgesehen sein.

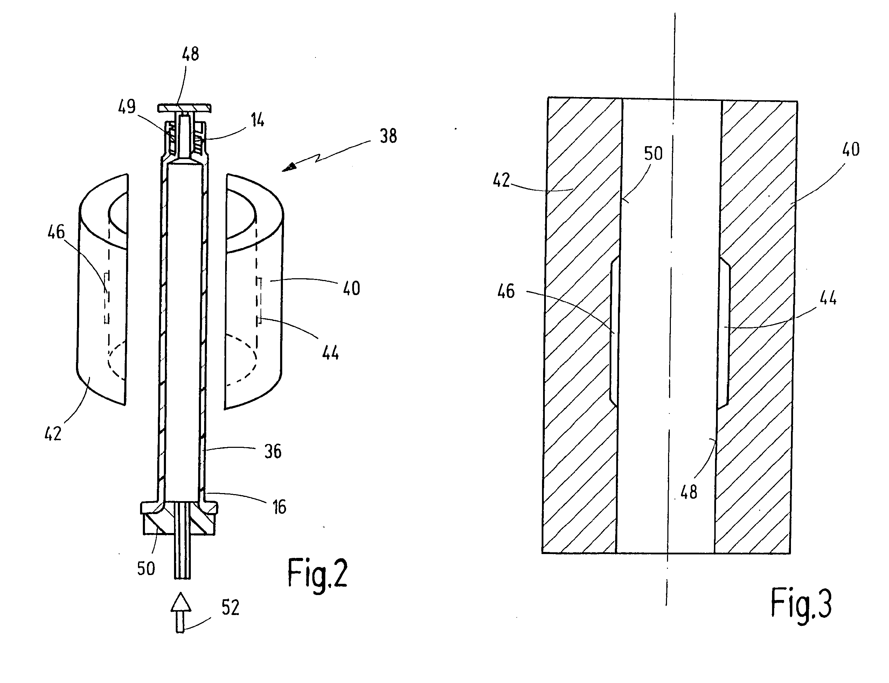

Die erfindungsgemäße Herstellung einer derartigen Mehrkammer-Spritze wird nunmehr anhand der Figuren 2 und 3 näher erläutert.

Zunächst wird aus einem geeigneten thermoformbaren Kunststoffmaterial ein Vorformling 36 hergestellt. Der Vorformling 36 ist rohrförmig, vorzugsweise zylindrisch ausgebildet und weist bereits das erste Ende 14 mit einer Ausflussspitze und das zweite Ende 16 mit einer Handhabe etwa in Form eines Fingerflansches auf. Am ersten Ende ist in Fig. 2 beispielhaft ein Luer-Lock-Gewinde 49 dargestellt, das das spätere Aufschrauben einer Injektionsnadel erlaubt. Der Vorformling 36 wird in grundsätzlich bekannter Weise vorzugsweise durch Spritzgießen in einer geeigneten Form hergestellt. Bei dem verwendeten Kunststoffmaterial kann es sich bspw. um ein zyklisches Olefin-Copolymer handeln, wie etwa COC 6013, das von der Firma Topas Advanced Polymers GmbH, Industriepark Höchst, Gebäude F 821, 65926 Frankfurt, Deutschland, vertrieben wird.

Zur Ausformung des mindestens einen Bypasskanals wird der Vorformling 36 innerhalb einer Matrize 38 platziert, in der der oder die Bypasskanäle als Vertiefungen an der Innenoberfläche ausgebildet sind. Zuvor wird der zu verformende Bereich partiell erwärmt und somit in den Warmformbereich des verwendeten Kunststoffmaterials gebracht. In diesem thermoelastischen Bereich ist das Material in sich noch formstabil, aber nicht mehr fest. Durch die gleichzeitige Anwendung einer Druckdifferenz erfolgt nunmehr eine Ausformung des mindestens einen Bypasskanals unter Druckeinfluss, so dass sich das Kunststoffmaterial an die Vertiefungen 44, 46 der Matrize 38 anlegt und so den mindestens einen Bypasskanal ausbildet.

Die verwendet Matrize 38 kann bspw. aus Werkstoffstahl bestehen und ist vorzugsweise zweiteilig mit einer ersten Matrizenhälfte 40 und einer zweiten Matrizenhälfte 42 ausgebildet, in denen die jeweiligen zu formenden Bypasskanäle als Vertiefungen 44, 46 (Negativform der Bypasskanäle) an der jeweiligen Innenoberfläche 48, 50 ausgebildet sind (vgl. Fig. 3).

Die verwendete Temperatur hängt vom verwendeten Kunststoffmaterial ab und kann grundsätzlich zwischen etwa 100°C bei tiefschmelzenden Kunststofftypen liegen und bis zu etwa 250°C bei Hochtemperaturtypen betragen. Gleichfalls abhängig vom verwendeten Kunststoffmaterial sind der erforderliche Druck und die Verformungszeit.

Wird bspw. das zuvor erwähnte Material COC 6013 verwendet, so beträg für eine Spritze von 5 ml Fassungsvermögen die Wandstärke des Spritzenkörpers 36 allgemein etwa 1,35 mm, im Bypassbereich etwa 1,0 mm. Zur Erzeugung der beiden Bypasskanäle wird der Vorformling 36 auf etwa 170°C erwärmt und mit einem Pressdruck von etwa 3 bis 4 bar von innen her beaufschlagt, wozu das erste Ende 14 durch einen Stopfen 48 verschlossen wird und das zweite Ende 16 gleichfalls durch einen Stopfen 50 verschlossen wird und der Druck gemäß dem Pfeil 52 durch einen geeigneten Zuführkanal zugeführt wird. Die Druckdauer beträgt etwa 2 Sekunden.

Die Abmessungen der verwendeten Bypasskanäle sind vom Format der Spritze abhängig. Bei einer Spritze von 5 ml Fassungsvermögen werden in der Regel folgende Maße verwendet:
- Kanaltiefe 0,4 bis 1 mm;
- Kanallänge so kurz wie möglich, aber länger als das Trennelement 28, bspw. 6 bis 12 mm.
- Kanalbreite etwa 1 bis 4 mm.

Wie zuvor bereits erwähnt, kann die Kanalform variieren. Auch die Anzahl der Kanäle kann variieren und kann von der Art der zu vermischenden Substanzen (z.B. deren Viskosität) beeinflusst sein. Insgesamt werden die Kanäle so kurz wie möglich gehalten, um nicht zu viel Totvolumen für die zu vermischenden Substanzen zu erzeugen.

## Patentansprüche

1. Verfahren zur Herstellung einer Mehrkammer-Spritze (10) mit Bypasskanal (22) aus Kunststoff mit folgenden Schritten:
- Herstellen eines rohrförmigen Vorformlings (36), vorzugsweise durch Spritzgießen, aus einem thermoformbaren Kunststoff;
- Bereitstellen einer Matrize (38), die zum Umschließen des Vorformlings (36) von außen in einem Bereich eines zu bildenden Bypasskanals (22) ausgebildet ist, wobei der zumindest eine Bypasskanal (22) als Vertiefung (44, 46) an einer Innenoberfläche (48, 50) der Matrize (38) ausgebildet ist;
- Erwärmen des Vorformlings (36) bis auf eine Temperatur oberhalb des Erweichungsbereiches des Kunststoffes, vorzugsweise innerhalb des Warmformbereiches, und Platzieren in einem Bereich innerhalb der Matrize (38) an einer vorbestimmten axialen Position;
- Beaufschlagen des Vorformlings (36) mit einer Druckdifferenz, bis der mindestens eine Bypasskanal (22) plastisch ausgeformt ist; und
- Abkühlen des so hergestellten Spritzenkörpers auf Raumtemperatur.

2. Verfahren nach Anspruch 1, bei dem der Vorformling (36) zur Ausformung des mindestens einen Bypasskanals (22) mit einem Überdruck von innen beaufschlagt wird.

3. Verfahren nach Anspruch 2, bei dem der Vorformling (36) zur Ausformung des mindestens einen Bypasskanals (22) mit einem Überdruck von mindestens 1 bar, vorzugsweise von mindestens 1,5 bar, weiter bevorzugt von mindestens 2 bar, vorzugsweise von mindestens 2,5 bar beaufschlagt wird.

4. Verfahren nach Anspruch 2 oder 3, bei dem der Vorformling (36) zur Ausformung des mindestens einen Bypasskanals (22) mit einem Überdruck von höchstens 10 bar, vorzugsweise von höchstens 8 bar, weiter bevorzugt von höchstens 5 bar, vorzugsweise von höchstens 4 bar beaufschlagt wird.

5. Verfahren nach einem der vorhergehenden Ansprüche, bei dem die Beaufschlagung des erwärmten Vorformlings (36) mit einer Druckdifferenz über eine Zeit zwischen 0,5 und 10 Sekunden, vorzugsweise zwischen 1 und 5 Sekunden erfolgt, um mindestens einen Bypasskanal (22) bei einer Wanddicke von 0,5 bis 2 mm im Bereich des Bypasskanals (22) auszuformen.

6. Verfahren nach einem der vorhergehenden Ansprüche, bei dem der mindestens eine Bypasskanal (22) bei einer Temperatur im Bereich von 120 bis 220 °C, vorzugsweise von 140 bis 200 °C, weiter bevorzugt von 160 bis 180 °C, besonders bevorzugt bei etwa 170 °C plastisch ausgeformt wird.

7. Verfahren nach einem der vorhergehenden Ansprüche, bei dem als Kunststoff ein lineares oder zyklisches olefinisches Polymer oder Copolymer verwendet wird.

8. Verfahren nach Anspruch 1, 6 oder 7, bei dem der Vorformling (36) zur Ausformung des mindestens einen Bypasskanals (22) mit einem Unterdruck von außen beaufschlagt wird.

9. Verfahren nach einem der vorhergehenden Ansprüche, bei dem der Vorformling (36) im Wesentlichen zylindrisch ausgebildet ist und ein erstes Ende (14) mit einer Verjüngung und ein zweites Ende (16) aufweist.

10. Verfahren nach Anspruch 9, bei dem am zweiten Ende (16) eine Handhabe (18), vorzugsweise in Form eines Flansches, angeformt ist.

11. Verfahren nach einem der vorhergehenden Ansprüche, bei dem der Vorformling (36) an seiner Innenoberfläche mit einem Gleitmaterial, vorzugsweise mit Silikonöl, beschichtet wird, bevor der mindestens eine Bypasskanal (22) erzeugt wird.

12. Verfahren nach einem der vorhergehenden Ansprüche, bei dem ein Trennelement (28) in den Spritzenkörper (12) derart eingeführt wird, dass eine erste Kammer (24) auf der Seite des ersten Endes (14) gebildet wird, die den mindestens einen Bypasskanal (22) einschließt und durch das Trennelement (28) von einer zweiten Kammer (26) auf der Seite des zweiten Endes (16) getrennt ist.

13. Verfahren nach Anspruch 12, bei dem die erste Kammer (24) mit einem ersten Medium befüllt wird und durch Aufsetzen eines Verschlusses (20) auf das erste Ende (14) verschlossen wird.

14. Verfahren nach Anspruch 12 oder 13, bei dem die zweite Kammer (26) mit einem zweiten Medium befüllt und mittels eines Kolbens (32) verschlossen wird, an dem ein Dichtelement (30) zur Abdichtung der zweiten Kammer (26) vorgesehen ist.

15. Verfahren nach einem der vorhergehenden Ansprüche, bei dem das Trennelement (28) und das Dichtelement (30) aus einem elastomeren Material bestehen .

16. Verfahren nach einem der vorhergehenden Ansprüche, bei dem eine Matrize (38) verwendet wird, die als zweiteilige Matrize, vorzugsweise bestehend aus zwei Formhälften (40, 42) ausgebildet ist.
